# EUROPÄISCHE PATENTSCHRIFT

(11) **EP 0 787 752 B1**
(45) Veröffentlichungstag und Bekanntmachung des Hinweises auf die Patenterteilung: **09.04.2003**
(21) Anmeldenummer: 97100855.2
(22) Anmeldetag: 21.01.1997
(51) Int. Cl.: C08G 18/79, C08G 18/72

(54) **Niedrigviskose Polyisocyanatmischung**
Low viscosity polyisocyanate mixture
Mélange de polyisocyanate à faible viscosité

(30) Priorität: 02.02.1996 DE 19603736
(43) Veröffentlichungstag der Anmeldung: 06.08.1997
(73) Patentinhaber: BAYER AG, 51368 Leverkusen (DE)
(72) Erfinder: Richter, Frank, Dr., 51373 Leverkussen (DE); Pedain, Josef, Dr., 51061 Köln (DE); Bock, Manfred, Dr., 51375 Leverkussen (DE); Halpaap, Reinhard, Dr., 51519 Odenthal (DE)

(56) Entgegenhaltungen:
- EP-A- 0 478 990
- EP-A- 0 693 512
- FR-A- 2 311 813

## Beschreibung

Die Erfindung betrifft niedrigviskose (Poly)Isocyanatmischungen.

Polyisocyanate, d.h. Isocyanate mit mindestens zwei NCO-Gruppen im Molekül, haben bei der Herstellung von Kunststoffen, Lacken und Beschichtungsmitteln auf Polyurethanbasis eine große wirtschaftliche Bedeutung. Wegen ihrer geringen Vergilbungsneigung unter Lichteinfluß kommt insbesondere den Vertretern, die ausschließlich (cyclo)aliphatisch gebundene NCO-Gruppen enthalten, eine hervorragende Bedeutung bei der Herstellung qualitativ hochwertiger Lackrohstoffe und Beschichtungsmittel zu.

Niedermolekulare aliphatische Diisocyanate wie z. B. Hexamethylendiisocyanat (HDI) oder Isophorondiisocyanat (IPDI) sind nur NCO-difunktionell und wegen ihres hohen Dampfdruckes aus arbeitshygienischen Gründen als solche nicht in den o.g. Anwendungen einsetzbar. Deshalb bedient man sich verschiedener Verfahren zur Modifizierung dieser Diisocyanate, wie z. B. der anteiligen Umsetzung mit zwei- und höherwertigen Alkoholen (Prepolymerisierung), der Biuretisierung sowie der Trimerisierung unter Ausbildung von Isocyanuratstrukturen. Eine Übersicht dieser Verfahren des Standes der Technik findet sich in: H. J. Laas, R. Halpaap und J. Pedain, J. Prakt. Chem. 1994, 336, 185 - 200.

Hierbei zeigte sich, daß für optimale Eigenschaften der aus diesen Polyisocyanaten hergestellten Lackrohstoffe und Beschichtungsmittel eine mittlere NCO-Funktionalität der Isocyanathärterkomponente im Bereich von 3 bis 4 nötig ist. Höherfunktionelle Produkte liefern keine wesentliche Verbesserung der mechanischen und chemischen Beständigkeiten mehr, während bei Funktionalitäten die deutlich unter 3 liegen sehr häufig Einbußen in der Qualität der Endprodukte, wie z. B. mangelhafte Lösungsmittelbeständigkeiten, hingenommen werden müssen.

Ein Nachteil, der allen Systemen des Standes der Technik anhaftet, die den o.g. Ansprüchen gerecht werden, ist die mitunter sehr hohe Viskosität, die den Einsatz von Lösungsmitteln wie z. B. Solventnaphtha, Xylol, Methoxypropylacetat usw. zu ihrer Verdünnung auf gut verarbeitbare Viskositäten nötig macht. Diese Lösungsmittel entweichen beim Aushärten des Lackes oder der Beschichtung und können, sofern sie nicht entsorgt, z. B. verbrannt werden, als leichtflüchtige organische Verbindungen (engl.: volatile organic compounds, VOC) die Umwelt belasten.

Aus diesem Grunde sind eine Reihe von Stoffen und Verfahren vorgeschlagen worden, die zur Viskositätserniedrigung von (Lack)polyisocyanat(mischung)en beitragen sollen, ohne die angesprochene VOC-Problematik zu verursachen. Zum einen sind dies sogenannte Reaktivverdünner, d.h. Stoffe, die eine niedrige Eigenviskosität, üblicherweise unter 200 mPa·s bei 23 °C, besitzen und gegenüber Reaktionspartnern der Polyisocyanate, z. B. Polyhydroxylverbindungen, reaktionsfähige Gruppen besitzen. Hierfür haben sich insbesondere Polyisocyanate aliphatischer Diisocyanate, wie z. B. HDI, mit Uretdionstruktur (sog. "Dimerisate") und Allophanatstruktur bewährt (H. J. Laas, R. Halpaap und J. Pedain, J. Prakt. Chem. 1994, 336, 196 - 198).

Dabei ist es für die Viskosität der Polyisocyanatmischung meist unerheblich, ob sie simultan durch gleichzeitige Bildung der höherviskosen, z. B. Isocyanurat- und niedrigerviskosen, z. B. Allophanat-Strukturanteile hergestellt wurde, oder ob separat hergestellte Produkte, die z. B. im wesentlichen aus reinen Isocyanurat-Polyisocyanaten und reinen AllophanatAllophanat-Polyisocyanaten bestehen, im nachhinein gemischt werden.

Sowohl Uretdiongruppen enthaltende, als auch Allophanat-Polyisocyanate, sofern sie auf der Basis difunktioneller Ausgangsisocyanate durch Umsetzung mit Monoalkoholen erhalten wurden, sind streng NCO-difunktionell. Allophanate auf Basis höherwertiger Alkoholmischungen hingegen weisen keine Viskositätsvorteile gegenüber Biuret- bzw. Isocyanurat-Polyisocyanaten auf (DE-A 2 729 990). Daraus resultiert zwangsläufig, daß die Funktionalität einer Polyisocyanatmischung durch eine wie auch immer geartete Kontaminierung mit o.g. Spezies erniedrigt wird. Wie die Vergleichsbeispiele 1 und 2 zeigen, sind für eine signifikante Viskositätserniedrigung bei HDI-Polyisocyanaten bereits derartig hohe Konzentrationen an difunktionellen Reaktivverdünnern nötig, daß die Funktionalität der resultierenden Mischung bereits deutlich unter 3 liegt.

Als Maß für eine signifikante Viskositätserniedrigung muß im System HDI-Trimerisat - Reaktivverdünner natürlich immer die literaturbekannte Viskosität des reinen Tris(6-isocyanatohexyl)isocyanurats verwendet werden (ca. 700 m·Pas bei 23 °C, vgl. Bsp. 3 sowie DE-A 3 810 908; WO-A 93/07 183). Dieses läßt sich bei sehr niedrigen Umsatzungsraten während der Trimerisierung von HDI als Hauptprodukt erhalten bzw. kann auch durch extraktive oder destillative Aufarbeitung von Oligomerenmischungen entsprechender Produkte, die mit einer höheren Umsetzungsrate aus monomerem HDI hergestellt werden, abgetrennt werden. Weder das eine noch das andere Verfahren ist jedoch unter wirtschaftlichen Aspekten von Vorteil, da einerseits die niedrige Umsetzungsrate einen enormen Verlust in der Harzausbeute und einen großen Energieaufwand, hauptsächlich verursacht durch die im Anschluß an die Trimerisierung nötige Monomerabtrennung nach sich zieht und andererseits Extraktions- bzw. Destillationsverfahren, neben den Mehrkosten des Verfahrens, den Zwangsanfall höherviskoser Fraktionen verursachen.

Entsprechendes gilt für Produkte, die aus anderen Diisocyanaten als HDI bzw. nach anderen Verfahren, als der Trimerisierung (Isocyanuratbildung) gewonnen werden. Beispielhaft genannt seien Urethan-Polyisocyanate ("Prepolymere"), Biurete oder Allophanate.

Andererseits lassen sich niedrigviskose aliphatische Polyisocyanate mit optimaler Funktionalität auch durch alternative Aufbaureaktionen, z. B. durch Umsetzung silylierter Alkohole mit Isocyanatoalkansäurechloriden herstellen (Ch. Zwiener, L. Schmalstieg, M. Sonntag, K. Nachtkamp, und J. Pedain, Farbe und Lack, 1991, 1052 - 1057 und darin zit. Literatur). Nachteilig hierbei ist, daß Isocyanatoalkansäurechloride technisch nicht zur Verfügung stehen und in ihrer Handhabung problematisch sein können. Das Verfahren ließe sich nur mit einem hohen technischen Aufwand realisieren, der durch die erwarteten Vorteile der Produkte, im wesentlichen die niedrige Viskosität der Polyisocyanate, nicht aufgewogen wird.

Aufgabe der vorliegenden Erfindung war es, eine Polyisocyanatmischung zur Verfügung zu stellen, die neben den bewährten Eigenschaften der Produkte des Standes der Technik, insbesondere der Isocyanurat-Polyisocyanate, eine deutlich verringerte Viskosität aufweisen. Dabei sollte die NCO-Funktionalität der erfindungsgemäßen Polyisocyanate im Bereich der bewährten, qualitativ hochwertigen Produkte des Standes der Technik, in jedem Fall jedoch über 3 liegen.

Diese Aufgabe konnte durch Mischung eines höherviskosen (η > 1000 mPa·s bei 23°C) Polyisocyanates, beispielsweise auf Isocyanuratbasis ("Trimerisat"), mit einem NCO-trifunktionellen (cyclo)aliphatischen Polyisocyanat des Molekulargewichtsbereichs von 200 bis 600 g/mol gelöst werden. Derartige (cyclo)aliphatische Triisocyanate sind beispielsweise nach der Lehre der DE-A 3109276 C 1 durch Phosgenierung der entsprechenden Triamine zugänglich und lassen sich, obwohl sie sehr hochsiedend sind, auf destillativem Wege reinigen. Ihrem alleinigen Einsatz, beispielsweise als Härterkomponente in Polyurethanlacken stehen allerdings ihr hoher NCO-Gehalt der zwangsläufig in einer nur marginalen Reduzierung des VOC-Wertes der Lackformulierung resultiert, der noch nicht niedrig genug liegende Dampfdruck sowie eine Reihe von Nachteilen entgegen, die sich bei der Formulierung der gebrauchsfertigen Lackmischung offenbaren, z. B. kurzes Potlife, nicht optimale Trocknung, stärkere Vergilbung der erhaltenen Lackfilme etc. (vgl. M. Ojunga-Andrew, H.P. Higginbottom und L.W. Hill, Waterborne, Higher Solids and Powder Coatings Symposium, Febr. 22-24, 1995, New Orleans, S. 200-210).

Gegenstand der vorliegenden Erfindung sind Polyisocyanatmischungen, bestehend aus 50 bis 98 Masse-% eines konventionellen Polyisocyanates mit einer dynamischen Viskosität im Bereich von 0,7 bis 15 Pa·s bei 23 °C und 2 bis 50 Masse-% eines (cyclo)aliphatischen Triisocyanates mit einer Molmasse im Bereich von 200 bis 600 g/mol und einer dynamischen Viskosität unter 200 mPa·s bei 23°C, dadurch gekennzeichnet, daß die Polyisocyanatmischungen die halbe Viskosität der höherviskosen Polyisocyanatkomponente bereits bei weniger als 30 Masse-%, vorzugsweise weniger als 20 Masse-%, Anteil der niedrigerviskosen Komponente aus der Gruppe der genannten Triisocyanate aufweist.

Ein weiterer Gegenstand der Erfindung ist die Verwendung der Isocyanatmischungen ggf. in blockierter Form, zur Herstellung von Lacken und Beschichtungen.

Der Erfindung liegt die überraschende Beobachtung zugrunde, daß sich die Viskosität von Polyisocyanat(mischung)en bereits durch Zusatz sehr geringer Mengen an Triisocyanato(cyclo)alkanen des Molekulargewichtsbereiches von 200 bis 600 g/mol drastisch reduzieren läßt, wohingegen die Beimengung bekannter Reaktivverdünner des Standes der Technik, die ebenfalls eine sehr niedrige Eigenviskosität (≤ 200 mPa·s bei 23 °C) aufweisen, in einer nur sehr langsamen Abnahme der Viskosität bei zunehmender Verdünnung der höherviskosen Polyisocyanatkomponente mit dem Reaktivverdünner resultiert. Hinzu kommt, daß die bekannten Reaktivverdünner des Standes der Technik weniger als NCO-trifunktionell sind und ihre Abmischungen mit höherfunktionellen Polyisocyanaten, deren Funktionalität bei Viskositäten zwischen 0,7 und 15 Pa·s im Bereich von 3 bis etwa 4,5 liegt, in der Regel bereits dann den Funktionalitätswert 3 erreicht bzw. unterschritten haben, wenn erst die Viskosität der jeweiligen - in einigen Fällen aus der Literatur bekannten - idealen, trifunktionellen Komponente des betreffenden Polyisocyanates erreicht wurde (Vergleichsbeispiele 1 bis 3).

Demgegenüber lassen sich durch die erfindungsgemäße Beimischung aliphatischer Triisocyanate des Molekulargewichtsbereiches von 200 bis 600 g/mol mit nur geringen Anteilen dieser Verbindungen im Polyisocyanatgemisch bereits drastische Viskositätserniedrigungen bei nur unwesentlich verringerter Funktionalität der Polyisocyanatmischung erzielen.

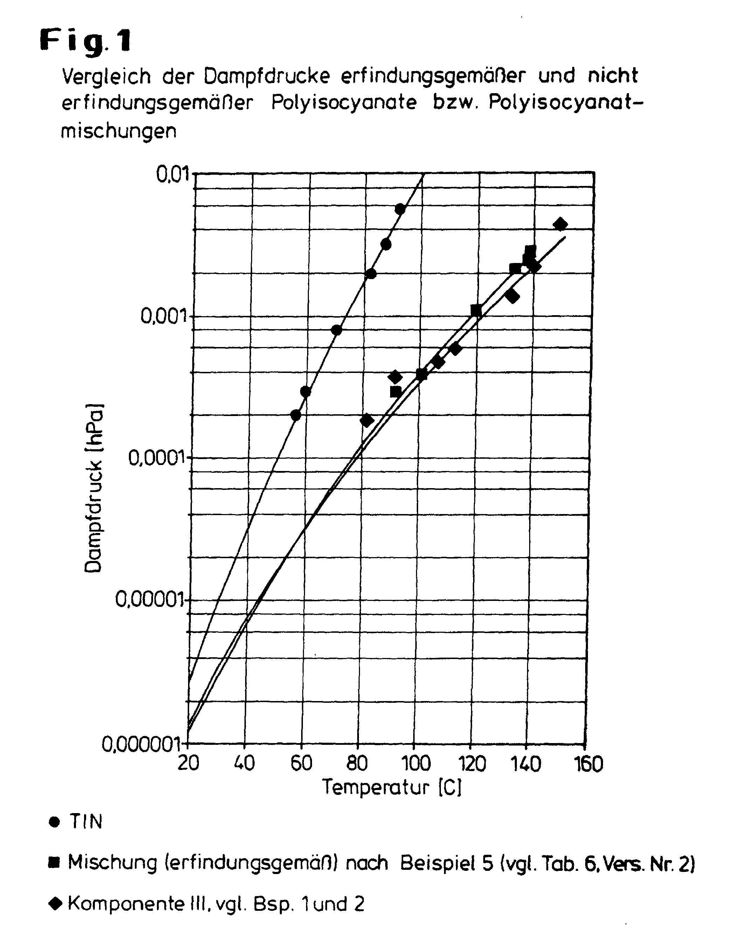

Die erfindungsgemäßen Polyisocyanatmischungen zeichnen sich sowohl durch ihre geringe Viskosität als auch durch einen niedrigen Dampfdruck aus, der von der Triisocyanatkomponente des Molekulargewichtsbereiches von 200 bis 600 g/mol allein noch nicht erreicht wird, was deren alleinigem Einsatz als NCO-Härterkomponente aus arbeitshygienischer Sicht im Wege stehen könnte (vgl. Abb. 1).

Zur Bereitstellung der erfindungsgemäßen Polyisocyanatmischungen ist es dabei belanglos, ob die Abmischung der höher- und der niedrigviskoseren Komponenten als letzter Verfahrensschritt erfolgt, oder ob die Vermischung in einem vorgeschalteten Schritt, beispielsweise bei der Herstellung der höherviskosen Komponente, erfolgt. So kann es bei genügender Siedepunkts- bzw. Verdampfungsenthalpiedifferenz zwischen der, zur Herstellung der höherviskosen Polyisocyanatkomponente eingesetzten, monomeren Verbindung(en) (in der Regel einem Diisocyanat bzw. einer Diisocyanatmischung), und dem Siedepunkt bzw. der Verdampfungsenthalpie des niedrigviskosen Triisocyanato(cyclo)alkans des Molgewichtsbereiches von 200 bis 500 g/mol durchaus von Vorteil sein, letztere vor einer destillativen Abtrennung des erstgenannten Monomers aus einer rohen Oligomerisatmischung, wie sie beispielsweise bei der anteiligen Trimerisierung oder auch Biuretisierung monomerer Diisocyanate anfallen, beizumischen. Hierdurch gelingt es beispielsweise, an sich sehr hochviskose Harze, beispielsweise auf Basis cycloaliphatischer Diisocyanate, bei so niedrigen Temperaturen destillativ von anhaftendem monomeren Diisocyanat zu befreien, wie es der Siedepunkt bzw. die Verdampfungsenthalpie dieser Verbindung gerade gestatten und nicht, wie es bei einigen Systemen des Standes der Technik der Fall ist, an bestimmte Mindesttemperaturen durch die (Schmelz)viskosität des resultierenden Harzes gebunden zu sein.

Selbstverständlich können die erfindungsgemäßen niedrigviskosen Polyisocyanatmischungen auch in Abmischung mit weiteren Stoffen verwendet werden, wie z.B. den weiter oben genannten Reaktivverdünner des Standes der Technik, obwohl dies nicht bevorzugt ist, sowie Lacklösungsmitteln oder Lacklösungsmittelgemischen wie beispielsweise Toluol, Xylol, Cyclohexan, Chlorbenzol, Butylacetat, Ethylacetat, Ethylglykolacetat, Methoxypropylacetat, Aceton, Testbenzin, höher substituierte Aromaten (Solventnaphtha®, Solvesso®, Shellsol®, Isopar®, Nappar®, Diasol®). Weiterhin können auch Hilfs- und Zusatzmittel verwendet werden, wie z.B. Benetzungsmittel, Verlaufsmittel, Hautverhinderungsmittel, Antischaummittel, Mattierungsmittel, viskositätsregulierende Stoffe, Pigmente, Farbstoffe, UV-Absorber und Stabilisatoren gegen thermische und oxidative Einflüsse.

Als Komponenten zur Bereitstellung der erfindungsgemäßen niedrigviskosen Polyisocyanatmischung eignen sich einerseits (als höherviskose Komponente) prinzipiell alle Polyisocyanate des Standes der Technik, z. B. solche auf Urethan-("Prepolymer"), Biuret- sowie Trimerisatbasis (Isocyanurate), aber auch höherviskose Produkte vom Allophanattyp, wie sie beispielsweise bei der Allophanatisierung aliphatischer Diisocyanate mit zwei- und höherwertigen Alkoholen, z. B. nach der Lehre der DE-A 2 729 990, erhalten werden können. Basieren diese Verbindungen auf Hexamethylendiisocyanat (HDI), ist eine Viskosität von ca. 700 mPa·s bei 23°C und einer NCO-Funktionalität ≥ 3 nicht wesentlich zu unterschreiten. Spezies der o.g. Strukturtypen auf Basis cycloaliphatischer Diisocyanate wie Isophorondiisocyanat (IPDI) oder Bis(isocyanatocyclohexyl)methan (Desmodur® W) sind bei Zimmertemperatur in der Regel fest oder hochviskose Öle ≥ 100 Pas).

Als niedrigviskose Triisocyanatkomponente des Molekulargewichtsbereiches von ca. 200 bis 600 g/mol eignen sich prinzipiell alle Spezies der Formel (I) 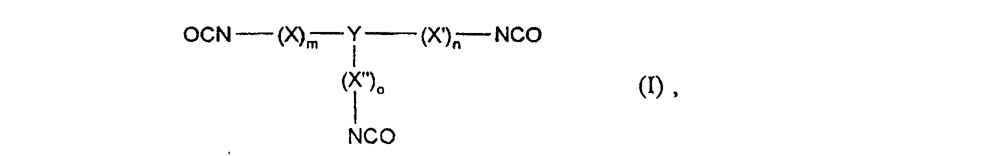 in denen X, X' und X" für gleiche oder verschiedene Reste wie:
- CR₂-(R = H und/oder C₁-C₁₂-Alkyl), -O-, -S-, -N(R)-(R = C₁-C₂₀-Alkyl), -S(O)-,
- S(O)₂-,
- O-S(O)₂-, -O-S-(O)₂-O-
sowie zweiwertige, vier- bis zwölfgliedrige, bevorzugt sechsgliedrige, ggf. polycyclische cycloaliphatische Reste, die ggf. weitere Substituenten wie z. B. Isocyanatgruppen, C₁-C₂₀-Alkylgruppen sowie deren heteroatom (O, N, S) substituierte Analoga tragen können, Y ein beliebiger dreiwertiger Rest wie z. B. CH; N; C₄-C₂₀-(ggf. Poly)cycloalkyl ist und m, n sowie o für gleiche oder verschiedene ganze Zahlen aber auch Null stehen können, wobei die Summe dieser drei Zahlen 6 bis 20 betragen kann.

Bevorzugt werden Verbindungen eingesetzt, in denen X, X' und X" für Methylengruppen, CH₂, stehen, Y eine Methinverknüpfung, CH, darstellt und die Summe aus m, n und o zwischen 8 und 12 liegen kann.

Diese Triisocyanate lassen sich aus den ihnen zugrundeliegenden Triaminen, ggf. nach deren Überführung in ein leichter der Phosgenierung zugängliches Derivat wie z. B. ein (Tris)hydrochlorid, -Carbaminat o.ä., durch ein Phosgenierverfahren des Standes der Technik, z. B. nach der Lehre der DE-A 3 109 276 C 1, gewinnen. Sie können aber auch auf phosgenfreiem Wege synthetisiert werden, beispielsweise wie in: M. Ojunga-Andrew, H.P. Higginbottom und L.W. Hill, Waterborne, Higher Solids and Powdor Coatings Symposium, Febr. 22-24, 1995, New Orleans, S. 200-210, sowie darin zit. Literatur beschrieben wird. Sie fallen dabei als hochsiedende, nahezu geruchlose Flüssigkeiten mit einem NCO-Gehalt zwischen 63 und 21 Masse-% an.

In den nachstehend aufgeführten Beispielen beziehen sich alle Prozentangaben, wenn nicht anders vermerkt, auf die Masse. Die dynamischen Viskositäten wurden bei 23 °C mit einem Rotationsviskometer "Rheomat 115" der Fa. Contraves aufgenommen. Durch Messungen bei unterschiedlichen Schwergeschwindigkeiten wurde sichergestellt, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die Bestimmung der Dampfdrucke der erfindungsgemäßen Polyisocyanatmischungen und der Vergleichsprodukte erfolgte durch Extrapolation mit Hilfe der Antoine-Gleichung auf eine Temperatur von 20 °C nach der in der EG-Richtlinie 92/69/EWG, Anhang A4 festgelegten Methode.

In allen Beispielen werden die erfindungswesentlichen Messungen und Beobachtungen exemplarisch am Beispiel von HDI-Polyisocyanaten vom Isocyanurattyp ("Trimerisate") als höherviskose Komponente und Triisocyanatononan (4-Isocyanatomethyl-1,8-oktandiisocyanat, "TIN") als der niedrigerviskosen Komponente dargestellt. Dies erfolgt nur aus Gründen der besseren Vergleichbarkeit der erhaltenen Ergebnisse und bedeutet keine Beschränkung der Erfindung auf diese Produkte. Entsprechendes gilt für Mischungen auf Basis anderer (Poly)isocyanate und/oder anderer Strukturtypen, als der Isocyanurat-Polyisocyanate ("Trimerisate").

### Beispiele

### Beispiel 1 (Vergleichsbeispiel)

Ein HDI-Isocyanurat-Polyisocyanat mit einer mittleren NCO-Funktionalität von 3,2, einer dynamischen Viskosität bei 23 °C von 1455 ± 10 mPa·s sowie einem Equivalentgewicht von 187 g/val (Komponente I) wird mit genau eingewogenen Mengen eines niedrigviskosen Reaktivverdünners der
a) im wesentlichen aus einem HDI-Uretdion-Polyisocyanat mit einer mittleren NCO-Funktionalität von 2,5 (durch anteilig enthaltenes Trimerisat), einer dynamischen Viskosität bei 23 °C von ≤ 200 mPa·s sowie einem Equivalentgewicht von 187 g/val (Komponente II)
oder
b) im wesentlichen aus einem HDI-Allophanat-Polyisocyanat (Allophanatbasis = n-Butanol) mit einer mittleren NCO-Funktionalität von 2,0, einer dynamischen Viskosität bei 23 °C von ≤ 200 mPa·s sowie einem Equivalentgewicht von 264 g/val (Komponente III) besteht
abgemischt. Anschließend wird die Viskosität der homogenen, klaren Polyisocyanatmischungen gemessen. Die Ergebnisse sind in Tabelle 1 und 2 dargestellt.

**Tabelle 1**

| Nr. | Anteil Komponente I | Anteil Komponente II | η²³ |
|---|---|---|---|
| 1 | 100 | - | 1446 |
| 2 | 90 | 10,0 | 1185 |
| 3 | 80,14 | 19,86 | 898 |
| 4 | 60,05 | 39,95 | 599 |
| 5 | 40,04 | 59,96 | 399 |

**Tabelle 2**

| Nr. | Anteil Komponente I | Anteil Komponente III | η²³ |
|---|---|---|---|
| 1 | 100 | - | 1465 |
| 2 | 94,99 | 5,01 | 1346 |
| 3 | 89,96 | 10,04 | 1217 |
| 4 | 84,99 | 15,01 | 1097 |
| 5 | 80,01 | 19,99 | 937 |
| 6 | 70,2 | 29,98 | 818 |
| 7 | 60,02 | 39,98 | 658 |
| 8 | 50,07 | 49,93 | 539 |
| 9 | 19,98 | 80,02 | 300 |

Die Herstellung der vorstehend genannten Reaktivverdünner ist in der DE-OS 1 670 720 (Uretdione) bzw. DE-A 2 729 990 (Allophanate) vorbeschrieben.

### Beispiel 2 (Vergleichsbeispiel)

Ein HDI-Isocyanurat-Polyisocyanat mit einer mittleren NCO-Funktionalität von 3,5, einer dynamischen Viskosität bei 23 °C von 3720 ± 100 mPa·s sowie einem Equivalentgewicht von 195 g/val (Komponente IV) wird mit genau eingewogenen Mengen eines niedrigviskosen Reaktivverdünners der
a) im wesentlichen aus einem HDI-Uretdion - Polyisocyanat mit einer mittleren NCO-Funktionalität von 2,5 (durch anteilig enthaltenes Trimerisat), einer dynamischen Viskosität bei 23 °C von ≤ 200 mPa·s sowie einem Equivalentgewicht von 187 g/val (Komponente II)
   oder
b) im wesentlichen aus einem HDI-Allophanat-Polyisocyanat (Allophanatbasis = n-Butanol) mit einer mittleren NCO-Funktionalität von 2,0, einer dynamischen Viskosität bei 23 °C von ≤ 200 mPa·s sowie einem Equivalentgewicht von 264 g/val (Komponente III) besteht
abgemischt. Anschließend wird die Viskosität der homogenen, klaren Polyisocyanatmischungen gemessen. Die Ergebnisse sind in Tabelle 3 und 4 dargestellt.

**Tabelle 3**

| Nr. | Anteil Komponente IV | Anteil Komponente II | η²³ |
|---|---|---|---|
| 1 | 100 | - | 3720 |
| 2 | 90 | 10 | 2175 |
| 3 | 80,07 | 19,93 | 1915 |
| 4 | 58,87 | 41,13 | 998 |
| 5 | 50,07 | 49,93 | 758 |
| 6 | 20,02 | 79,98 | 319 |

**Tabelle 4**

| Nr. | Anteil Komponente I | Anteil Komponente II | η²³ |
|---|---|---|---|
| 1 | 100 | - | 3640 |
| 2 | 94,97 | 5,03 | 3112 |
| 3 | 90,05 | 9,95 | 2534 |
| 4 | 84,91 | 15,09 | 2294 |
| 5 | 79,87 | 20,13 | 1845 |
| 6 | 69,98 | 30,02 | 1456 |
| 7 | 60,13 | 39,87 | 1077 |
| 8 | 50,25 | 49,75 | 798 |
| 9 | 19,86 | 80,14 | 350 |

Wie den in Tabelle 1 bis 4 dargestellten Meßergebnissen zu entnehmen ist, erfolgt bei sukzessiver Verdünnung der höherviskosen Polyisocyanatkomponenten I bzw. IV mit den Reaktivverdünnerkomponenten II bzw. III eine relativ langsame Viskositätsabnahme der Mischungen. Um die Viskosität der idealen Isocyanurat-Trimerisatverbindung des HDI's zu erreichen (700 mPa·s, vgl. Beispiel 3), muß man in jedem Falle mindestens 30 % Reaktivverdünner zusetzen. Bei einer Viskosität von 700 mPa·s der nach Beispiel 1 bzw. 2 erhaltenen Mischungen liegt die NCO-Funktionalität der Produkte bei maximal 2,95 (Beispiel 1a) und fällt bis auf 2,58 ab (Beispiel 2b).

Wie im folgenden Beispiel gezeigt wird, ist diese Viskosität mit dem nahezu reinen HDI-Trimeren, Funktionalität n = 3, auch erreichbar, stellt mithin keinen Fortschritt gegenüber bestehenden Verfahren bzw. Systemen des Standes der Technik dar.

### Beispiel 3 (Vergleichsbeispiel)

1500 g der Komponente I aus Beispiel 1 werden bei einem Druck von 0,05 mbar bei einer Temperatur des Heizmediums von 220 °C der Dünnschichtdestillation in einem Kurzwegverdampfer unterworfen. Dabei gehen 267 g Destillat über, das anschließend bei 120°C / 0,05 mbar von monomerem HDI durch Dünnschichtdestillation befreit sind. Das so gereinigte Produkt weist eine dynamische Viskosität von 700 ± 10 mPa·s bei 23 °C auf und besteht nach Ausweis kombinierter analytischer Methoden (IR, NMR, GPC, MS) zu mehr als 98 % aus dem idealen Isocyanurat-Trimeren des Hexamethylendiisocyanates (1,3,5-Tris(6-isocyanatohexyl)isocyanurat).

Diese Messsungen stehen völlig im Übereinklang mit literaturbekannten Daten (vgl. WO-A 93/07 183, die in den dort angegebenen Beispielen zitierten Viskositäten wurden bei 25 °C an weniger reinen "Ideal-Isocyanurat"-Fraktionen gemessen).

### Beispiel 4 (erfindungsgemäß)

Ein HDI-Isocyanurat-Polyisocyanat mit einer mittleren NCO-Funktionalität von 3,2, einer dynamischen Viskosität bei 23 °C von 1455 ± 10 mPa·s sowie einem Equivalentgewicht von 187 g/val (Komponente I) wird mit genau eingewogenen Mengen an Triisocyanatononan ("TIN", 4-Isocyanatomethyl-1,8-oktandiisocyanat, NCO-Funktionalität = 3, dynamische Viskosität bei 23°C < 200 mPa·s, Equivalentgewicht = 84 g/val) abgemischt. Anschließend wird die Viskosität der homogenen, klaren Polyisocyanatmischungen gemessen (Tabelle 5).

**Tabelle 5**

| Nr. | Anteil Komponente I | Anteil TIN | η²³ |
|---|---|---|---|
| 1 | 100 | - | 1446 ± 10 |
| 2 | 89,24 | 10,76 | 678 |
| 3 | 79,77 | 20,23 | 379 |
| 4 | 70,45 | 29,55 | 219 |

### Beispiel 5 (erfindungsgemäß)

Ein HDI-Isocyanurat-Polyisocyanat mit einer mittleren NCO-Funktionalität von 3,5, einer dynamischen Viskosität bei 23°C von 3720 ± 100 mPa·s sowie einem Equivalentgewicht von 195 g/val (Komponente IV) wird mit genau eingewogenen Mengen an Triisocyanatononan ("TIN", 4-Isocyanatomethyl-1,8-oktandiisocyanat, NCO-Funktionalität = 3, dynamische Viskosität bei 23°C < 200 mPa·s, Equivalentgewicht = 84 g/val) abgemischt. Anschließend wird die Viskosität der homogenen, klaren Polyisocyanatmischungen gemessen (Tabelle 6).

Wie sofort ersichtlich ist, bedarf es zur Erniedrigung der Mischungsviskosität der erfindungsgemäßen Polyisocyanatmischungen nur eines Bruchteils an TIN im Vergleich zu den in Beispiel 1 und 2 aufgeführten Reaktivverdünnern des Standes der Technik.

**Tabelle 6**

| Nr. | Anteil Komponente I | Anteil Komponente II | η²³ |
|---|---|---|---|
| 1 | 100 | - | 3680 ± 20 |
| 2 | 90,01 | 9,99 | 1845 |
| 3 | 79,98 | 20,02 | 695 |
| 4 | 69,61 | 30,39 | 339 |

## Patentansprüche

1. Polyisocyanatmischungen aus 50 bis 98 Masse-% eines Polyisocyanat(gemisch)es mit einer dynamischen Viskosität im Bereich von 0,7 bis 15 Pas bei 23°C (Komponente A) und 2 bis 50 Masse-% eines (cyclo)aliphatischen Triisocyanat(gemisch)es mit einer Molmasse im Bereich von 200 bis 600 g/mol und einer dynamischen Viskosität unter 200 mPa·s bei 23 °C (Komponente B), **dadurch gekennzeichnet, daß** die Polyisocyanatmischungen aus A und B bereits bei weniger als 30 Masse-% Anteil der Komponente B die halbe Viskosität der Polyisocyanatkomponente A aufweisen.

2. Polyisocyanatmischungen gemäß Anspruch 1, **dadurch gekennzeichnet, daß** als Komponente A Produkte auf der Basis von Hexamethylendiisocyanat-Folgeprodukten wie z. B. Urethan-(Prepolymer-), Biuret-, Isocyanurat-(Trimerisat-) und/oder Allophanat- Polyisocyanate verwendet werden.

3. Polyisocyanatmischung gemäß Anspruch 1, **dadurch gekennzeichnet, daß** als Komponente B reine Verbindungen oder beliebige Mischungen von Spezies des Molekulargewichtsbereiches von ca. 200 bis 600 g/mol der Formel (I) 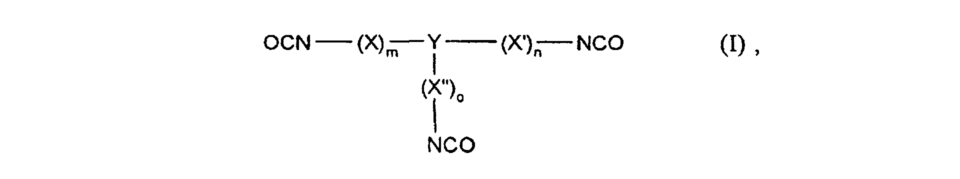 eingesetzt werden, in denen X, X' und X" für gleiche oder verschiedene Reste wie
-CR₂-(R = H und/oder C₁-C₁₂-Alkyl), -O-, -S-, -N(R)-(R = C₁-C₂₀-Alkyl), - S(O)-, -S(O)₂-, -O-S(O)₂-, -O-S-(O)₂-O- sowie zweiwertige, vier- bis zwölfgliedrige, ggf. polycyclische cycloaliphatische Reste, die ggf. weitere Substituenten, wie z. B. Isocyanatgruppen, C₁-C₂₀-Alkylgruppen sowie deren heteroatom (O, N, S) substituierte Analoga tragen können, Y ein beliebiger dreiwertiger Rest wie z. B. CH; N; C₄-C₂₀-(ggf. Poly)cycloalkyl ist, stehen und m, n sowie o für gleiche oder verschiedene ganze Zahlen aber auch Null stehen können, wobei die Summe dieser drei Zahlen 6 bis 20 betragen kann.

4. Die Verwendung der Polyisocyanatmischungen nach Anspruch 1 zur Herstellung von ggf. geschäumten, Polyurethankunst- sowie Klebstoffen, Lacken und Beschichtungsmitteln, wobei die NCO-Gruppen ggf. in blockierter Form vorliegen können.

## Claims

1. Polyisocyanate mixtures consisting of 50 to 98 mass-% of a polyisocyanate or a polyisocyanate mixture having a dynamic viscosity in the range from 0.7 to 15 Pa.s at 23°C (Component A) and 2 to 50 mass-% of a (cyclo)aliphatic triisocyanate or triisocyanate mixture having a molecular mass in the range from 200 to 600 g/mol and a dynamic viscosity below 200 mPa.s at 23°C (Component B), **characterised in that** the polyisocyanate mixtures consisting of A and B have half the viscosity of polyisocyanate Component A with a proportion of Component B as low as less than 30 wt-%.

2. Polyisocyanate mixtures according to Claim 1,
**characterised in that** by way of Component A use is made of products based on hexamethylenediisocyanate secondary products such as urethane (prepolymer) polyisocyanates, biuret polyisocyanates, isocyanurate (trimerisate) isocyanates and/or allophanate polyisocyanates.

3. Polyisocyanate mixture according to Claim 1,
**characterised in that** by way of Component B use is made of pure compounds or arbitrary mixtures of species of the molecular-weight range from about 200 to 600 g/mol of the formula (I) 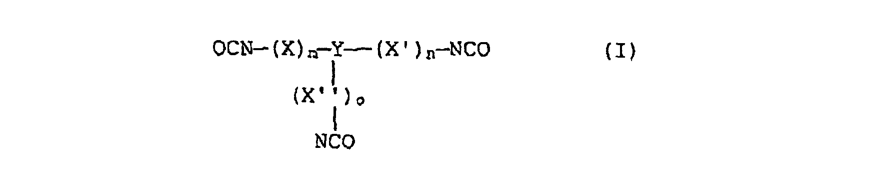 in which X, X' and X'' stand for the same or different residues such as:
-CR₂-(R = H and/or C₁-C₁₂-alkyl), -O-, -S-, -N(R)-(R = C₁-C₂₀- alkyl), -S(O)-, -S(O)₂-, -O-S(O)₂-, -O-S-(O)₂-O-as well as divalent, four- to twelve-membered, optionally polycyclic cycloaliphatic residues, which optionally may contain additional substituents such as isocyanate groups, C₁-C₂₀-alkyl groups and also their analogues substituted by heteroatoms (O, N, S), Y is an arbitrary trivalent residue such as CH; N; C₄-C₂₀-(optionally poly)cycloalkyl, and m, n and o may stand for the same or different integers but also for zero, whereby the sum of these three numbers may amount to between 6 and 20.

4. The use of the polyisocyanate mixtures according to Claim 1 for the production of optionally foamed, polyurethane synthetic substances and also adhesives, lacquers and coating agents, whereby the NCO groups may optionally be present in blocked form.

## Revendications

1. Mélanges de polyisocyanates consistant en 50 à 98 % en poids d'un polyisocyanate (ou mélange de polyisocyanates) à une viscosité dynamique dans l'intervalle de 0,7 à 15 Pa.s à 23°C (composant A) et 2 à 50 % en poids d'un triisocyanate (ou mélange de triisocyanates) (cyclo)aliphatique à une masse moléculaire dans l'intervalle de 200 à 600 g/mol et une viscosité dynamique inférieure à 200 mPa.s à 23°C (composant B) **caractérisés en ce que** les mélanges de polyisocyanates consistant en A et B présentent déjà une viscosité représentant la moitié de celle du composant polyisocyanate A à une proportion inférieure à 30 % en poids du composant B.

2. Mélanges de polyisocyanates selon la revendication 1, **caractérisés en ce que**, pour le composant A, on utilise des produits à base de dérivés de l'hexaméthylènediisocyanate, par exemple des polyisocyanate-uréthannes (prépolymère), des polyisocyanate-biurets, des polyisocyanate-isocyanurates (trimères) et/ou des polyisocyanate-allophanates.

3. Mélange de polyisocyanates selon la revendication 1, **caractérisé en ce que**, pour le composant B, on utilise des composés purs ou des mélanges quelconques de composés à des poids moléculaires dans l'intervalle d'environ 200 à 600 g/mol, répondant à la formule (I) 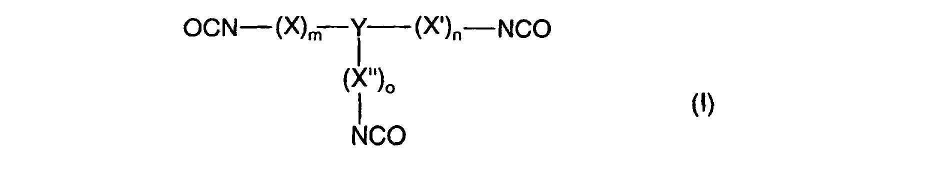 dans laquelle X, X' et X", ayant des significations identiques ou différentes, représentent par exemple
-CR₂- (R = H et/ou alkyle en C₁ à C₁₂), -O-, -S-, -N(R)- (R = alkyle en C₁ à C₂₀), -S(O)-, -S(O)₂-, -O-S(O)₂-, -O-S-(O)₂-O- ou encore des radicaux cycloaliphatiques divalents, de 4 à 12 chaînons, éventuellement polycycliques qui peuvent le cas échéant porter d'autres substituants, par exemple des groupes diisocyanate, des groupes alkyle en C₁ à C₂₀ et les groupes analogues à hétéroatomes (O, N, S), Y représente un chaînon trivalent quelconque, par exemple CH ; N ; cycloalkyle (éventuellement polycycloalkyle) en C₄ à C₂₀ ; m, n et o sont des nombres entiers identiques ou différents qui peuvent également être nuls, la somme des trois chiffres allant de 6 à 20.

4. Utilisation des mélanges de polyisocyanates selon la revendication 1 pour la préparation de résines synthétiques de polyuréthannes éventuellement gonflées en mousse, d'adhésifs, de peintures et de produits de revêtement, les groupes NCO étant éventuellement à l'état bloqué.
